# EUROPEAN PATENT SPECIFICATION

(11) **EP 4 513 502 B1**
(45) Date of publication and mention of the grant of the patent: **13.05.2026**
(21) Application number: 24194697.9
(22) Date of filing: 15.08.2024
(51) Int. Cl.: G16H 20/17, A47B 67/02, A61M 5/14, G07C 9/00, G16H 40/63, H04W 12/08, A61M 5/00, A61M 5/142

(54) **INTELLIGENT LOCK BOX FOR INFUSION DEVICE**
INTELLIGENTER VERSCHLUSSKASTEN FÜR EINE INFUSIONSVORRICHTUNG
BOÎTE DE VERROUILLAGE INTELLIGENTE POUR DISPOSITIF DE PERFUSION

(30) Priority: 25.08.2023 US 202363534781 P
(43) Date of publication of application: 26.02.2025
(73) Proprietor: CareFusion 303, Inc., San Diego, CA 92130 (US)
(72) Inventor: Langan, John, San Diego, 3750 (US); Workman, Michael K., San Diego, 3750 (US)
(74) Representative: Zenz Patentanwälte Partnerschaft mbB

(56) References cited:
- WO-A1-2023/122514
- CN-A- 115 316 819
- US-A1- 2020 394 602
- US-B1- 11 187 013

## Description

### BACKGROUND

This application relates generally to securable enclosures for infusion pumps.

A substance is a "controlled substance" when it is determined to be abusable, has shown a pattern of abuse leading to dependency, and that abuse has been shown to create a risk to public health and safety. In many instances, controlled substances have legitimate medical therapeutic uses, and so may be purchased, stored, dispensed and administered to patients during the treatment of injury or disease. Healthcare institutions may be required to maintain stores of controlled substances for these uses, and those stores may attract individuals who wish to use them for other purposes, leading to abuse. Those individuals may be employees of the healthcare institution itself. Thus, any organization that routinely uses controlled substances for valid therapeutic purposes can become a target for diversion.

Moreover, drug seeking behavior is highly adaptive and diverters will constantly challenge any control system looking for a way to defeat it and acquire the drugs they seek. While diverters may include patients or visitors of patients, diverters may also include healthcare professionals. Indeed, diversion of controlled substances by healthcare professionals can occur within any profession that has routine and appropriate access to these substances. The healthcare professionals with the most frequent access include physicians, nurses, pharmacists and pharmacy technicians. In general, when healthcare workers divert drugs, it is for personal use and is symptomatic of addiction. Also, drug diverters attempt to disguise their diversion as apparently legitimate use of controlled substances.

Current automated control systems have tightened security around controlled substances, but diversion still exists. While control systems limit the opportunity for diversion, they cannot entirely prevent it. Medical cabinets are routinely secured with locks and monitored. Lock boxes have been constructed and utilized for preventing access to the infusion pumps that deliver controlled substances. Lock boxes are designed to completely encompass, and prevent access to, both the infusion device and the medication container connected to the infusion pump. Lock boxes provide a good deterrent to diversion activities; however, a lock box must be manually removed, often with a physical key, before any adjustment can be made to the infusion pump, a time consuming process that imposes a significant impediment to critical care needs.

WO 2023/122514 A1 discloses a multi-function, modular pump system having a portable infusion pump configured for ambulatory use. The portable infusion pump can have a battery, a portable user interface, and a docking module or controller/container. The docking module or controller/container can have a security feature configured to prevent removal or unauthorized access and a pump mount configured to engage and stabilize the pump within the container, allow pump to maintain infusion continuity when removed, and enable rapid disengagement when authorized. An external user interface can allow for control of the contained pump, review of pump run status, log event history, settings, and configurations. A machine interface can allow control signals to pass from external interface to a contained, engaged portable pump. A simplified extending patient control can be configured to provide a bolus dose. The portable pump can be configurable for use in two modes: portable mode, and secured mode.

CN 115 316 819 A relates to the technical field of data displays, in particular to a data information display device based on genetic particle swarm optimization. Comprising a box body, a lifting device is arranged in the box body and comprises a first motor arranged on the inner bottom face of the box body, the output end of the first motor is connected with a rotating handle through a rotating shaft, the upper end of the rotating handle is sleeved with a moving block, the moving block is sleeved with a moving plate, and a first supporting rod is arranged above the moving plate and sleeved with a fixing rod; a fixing plate is arranged above the first supporting rod, two movable plates are inserted into the fixing plate, a first fixing block is arranged above the fixing plate, the first fixing block is connected with a telescopic plate through a first rotating column, the first fixing block is connected with a baffle through a second rotating column, a supporting device is inserted into the box body, steering wheels are arranged below the supporting device, and an intelligent switch is arranged in the box body. The data information display device based on the genetic particle swarm optimization has high-strength secrecy, the height can be adjusted through different platforms, and displacement is convenient.

### SUMMARY

The invention is defined in the independent claims. Preferable embodiments are further laid out in the dependent claims. The subject technology provides a method for securing a pump, comprising: securing at least a portion of an infusion pump within a lockbox, the lockbox configured to enclose and prevent access to at least a user interface of the infusion pump and a medication receptacle of the infusion pump, the lockbox comprising a lockbox control interface configured to obtain authorization for access to the lockbox from a pump communication interface of the infusion pump and enclosed within the lockbox; electronically coupling the lockbox control interface with the pump communication interface; facilitating transmission of a wireless credential to the pump communication interface for authorization to the lockbox; receiving, by the lockbox control interface from the pump communication interface, the authorization for access based on the wireless credential; and performing, by the lockbox control interface, an action according to the received authorization for access. Other aspects include corresponding devices and computer program products for implementation of the corresponding method and its features.

According to various aspects, a system for securing a pump, comprises: a lockbox configured to enclose and prevent access to at least a user interface of an infusion pump and a medication receptacle of the infusion pump; and a lockbox control interface associated with the lockbox configured to obtain authorization for access to the lockbox from a pump communication interface of the infusion pump and enclosed within the lockbox, the lockbox control interface comprising a processor configured to: electronically couple the lockbox control interface with the pump communication interface; facilitate transmission of a wireless credential to the pump communication interface for authorization to the lockbox; receive, from the pump communication interface, the authorization for access based on the wireless credential; and cause performance of an action according to the received authorization for access. Other aspects include corresponding devices, methods, and computer program products for implementation of the corresponding system and its features.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

For a better understanding of the various described implementations, reference should be made to the Description below, in conjunction with the following drawings. Like reference numerals refer to corresponding parts throughout the figures and description.
FIG. 1A depicts a first example patient care unit that includes a syringe pump mounted to a control unit, according to various aspects of the subject technology.
FIG. 1B depicts a second example of a patient care unit with connected functional modules, according to aspects of the subject technology.
FIG. 2A depicts a perspective view of the example lockbox enclosing the patient care unit.
FIG. 2B depicts a perspective view of an unlocking and opening of the lockbox of FIG. 2A, according to various aspects of the subject technology.
FIG. 2C depicts a front view of the lockbox in a closed and secure state, according to various aspects of the subject technology.
FIG. 2D depicts a font view of the lockbox in an open and unsecured state, according to various aspects of the subject technology.
FIG. 2E depicts a side view of the lockbox in a closed and secure state, according to various aspects of the subject technology.
FIG. 2F depicts a side view of the lockbox in an open and unsecured state, according to various aspects of the subject technology.
FIG. 2G depicts an exploded view of the lockbox 200, according to various aspects of the subject technology.
FIG. 3 depicts a system for securing an infusion device by authenticating a user to the infusion device via an intelligent lockbox, according to various aspects of the subject technology.
FIGS. 4 depicts an example process for securing an infusion device using an intelligent lockbox, according to aspects of the subject technology.
FIG. 5 is a conceptual diagram illustrating an example electronic system for securing an infusion device using an intelligent lockbox, according to aspects of the subject technology.

### DESCRIPTION

Reference will now be made to implementations, examples of which are illustrated in the accompanying drawings. In the following description, numerous specific details are set forth in order to provide an understanding of the various described implementations. However, it will be apparent to one of ordinary skill in the art that the various described implementations may be practiced without these specific details. In other instances, well-known methods, procedures, components, circuits, and networks have not been described in detail so as not to unnecessarily obscure aspects of the implementations.

The subject technology provides an intelligent system for securing an infusion device. As will be described further, the infusion device is secured within a lockbox, with the lockbox configured to enclose and prevent access to at least a user interface of the infusion pump and a medication receptacle of the infusion pump. The lockbox includes a communication interface configured to receive a credential and to pass that credential to the infusion device within the lockbox to obtain authorization for access to the lockbox from the infusion device.

According to various implementations, the lockbox comprises retractable multi-folding doors each configured to enclose respective portions of the infusion pump. Opening of the lockbox is motor controlled, and the lockbox includes a top configured to automatically open, and opposing sides of the housing that are configured to automatically slide in a rearward direction, to provide greater access to the infusion pump. When closed, the top, sides, and folding doors are integral to each other to seal the lockbox and prevent access to the infusion pump within the lockbox.

The lockbox of the subject technology may be communicatively connectable (e.g., using BLUETOOTH) to the infusion pump and/or a mobile device for receiving signals to open the lockbox. There is no need for a hard key, and clinicians may scan a badge to open the lockbox. On authentication, the mechanical parts of the lockbox (e.g., doors, top, sides) may open, with the doors folding and the sides sliding backwards to reveal the pump.

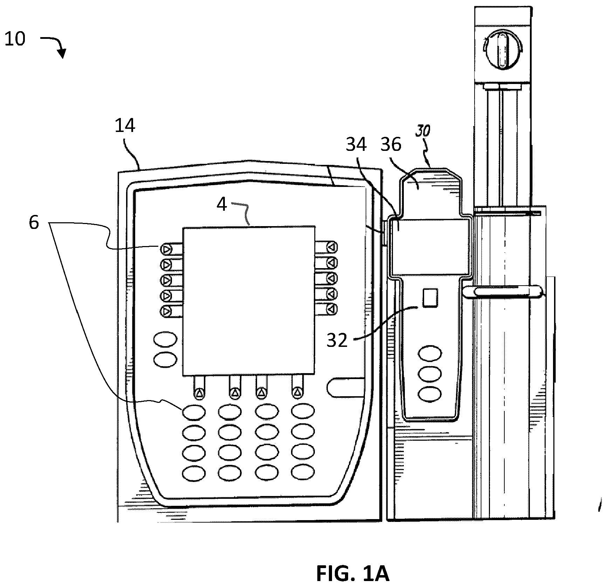

FIG. 1A depicts a first example patient care unit 10 that includes a syringe pump 30 mounted to a control unit 14, according to various aspects of the subject technology. According to various implementations, the control unit 14 may provide control and monitoring functionality for the syringe pump 30. In this regard, the control unit may include a display 4 for visually communicating various information, such as the operating parameters of a connected pump and alert indications and alert messages, and control keys 6 for selecting and/or setting control parameters and/or options for controlling connected modules such as syringe pump 30. The control unit 14 may also include a speaker to provide audible alerts. In some implementations, the display 4 may be implemented as a touchscreen display. In such implementations, the control keys 6 may be omitted or reduced in number by providing corresponding interactive elements via a graphical user interface presented via the display 4. In some implementations, each control key 6 may select a corresponding option displayed in display 4.

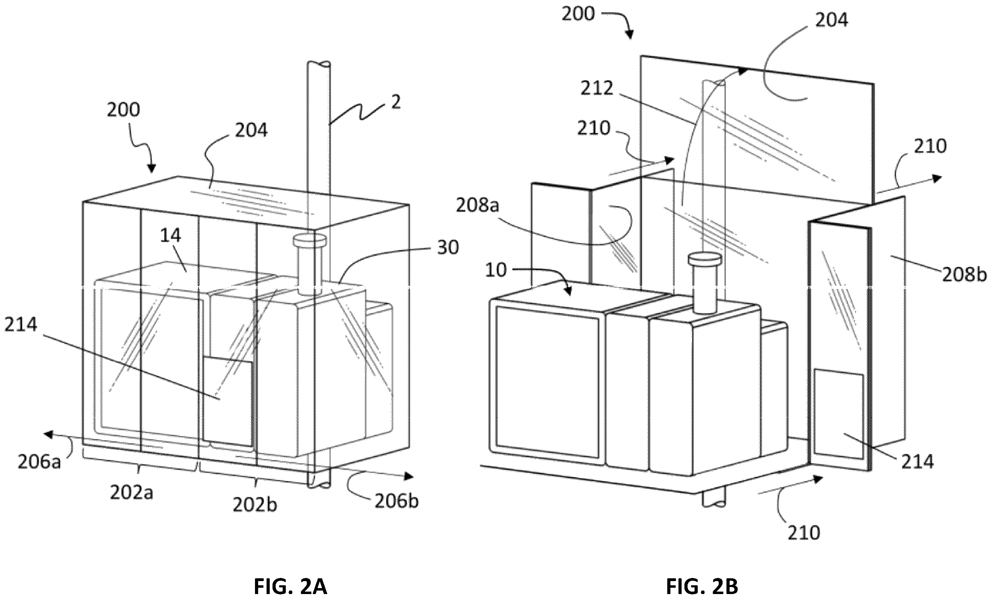

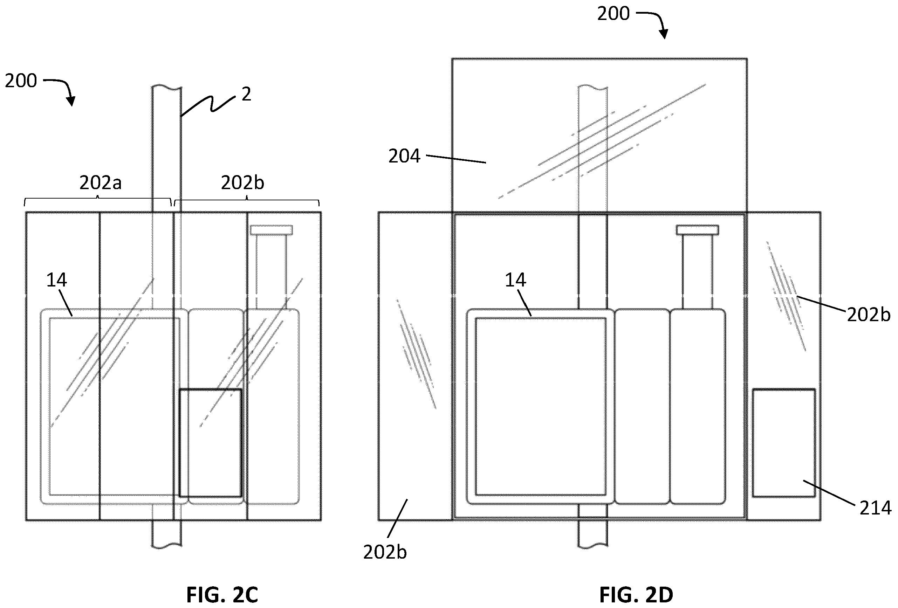

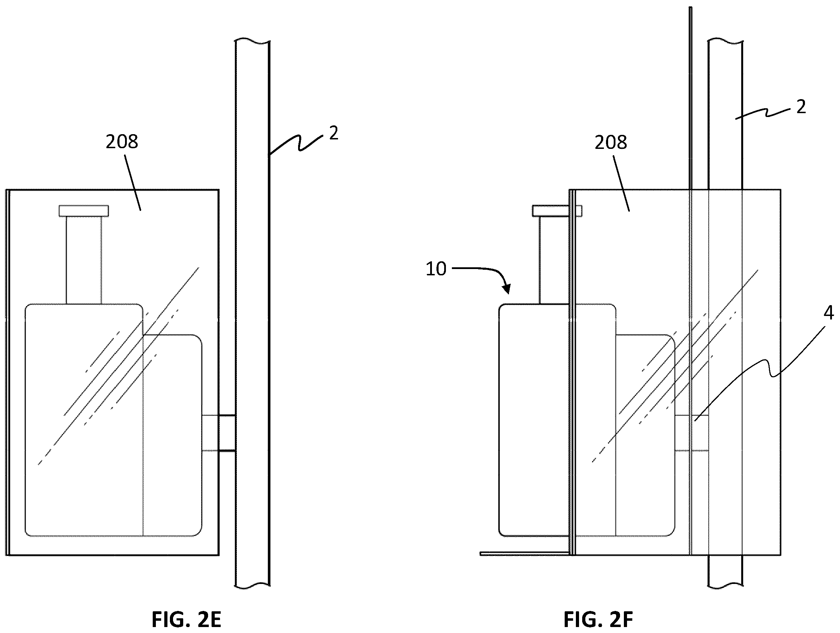

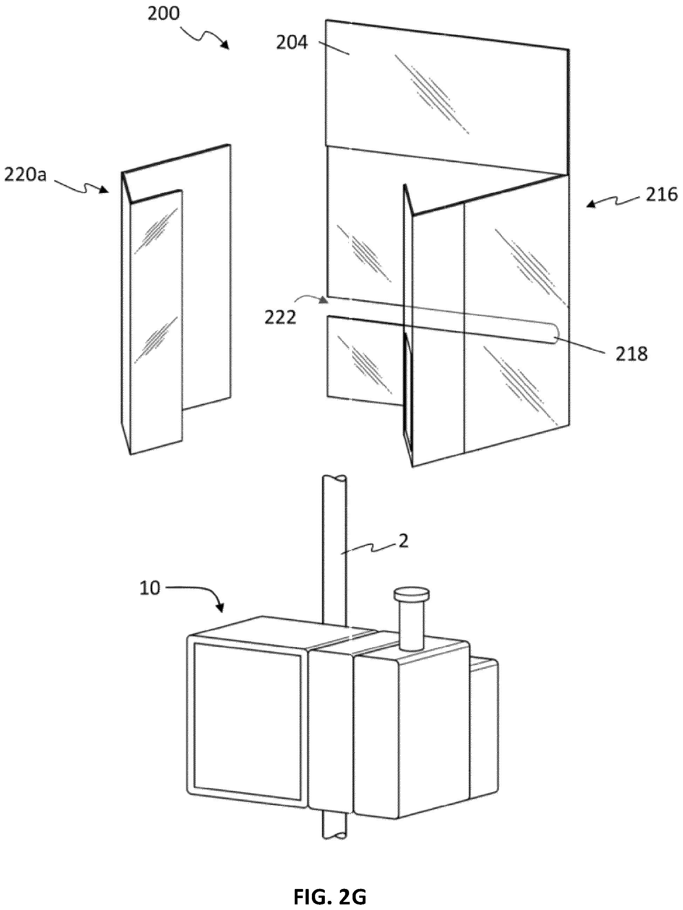

The syringe pump 30 can include a control panel 36 providing multiple buttons 32 for control of the pump 26 as well as a display 34 used to present pump-specific information to the operator (see also FIG. 2). The buttons 32 can allow the operator to program the pump 26 for the flow rate, the volume to be infused, and other pump parameters. The display 34 can present the programmed flow rate, the amount of fluid remaining to be infused, as well as alarms and other information.

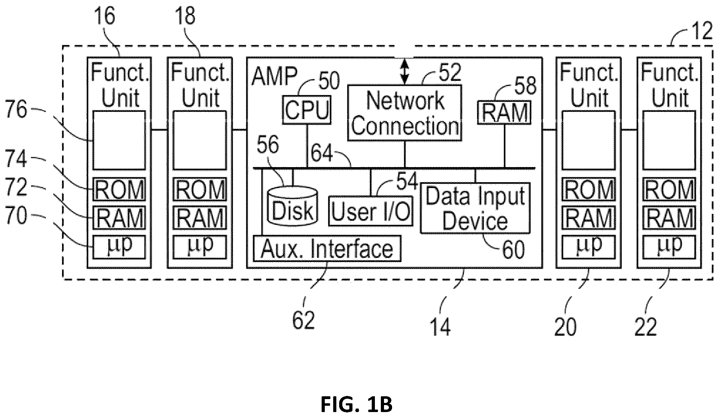

The control unit 14 may include a communications system (not shown) with which the control unit 14 may communicate with external equipment such as a medical facility server or other computer and with a portable processor, such as a handheld communication device or a laptop-type of computer, or other information device that a clinician may have to transfer information as well as to download drug libraries to a control unit (such as pump 30 or, e.g., modules of FIG. 1B). The communication module may be used to transfer access and interaction information for clinicians encountering the control unit or device coupled therewith (e.g., pump 30 or bar code scanner). The communications system may include one or more of a radio frequency (RF) system, an optical system such as infrared, a BLUETOOTH^{™} system, or other wired or wireless system. The bar code scanner and communications system may alternatively be included integrally with the infusion pump 30, such as in cases where a control unit is not used, or in addition to one with the control unit 14. Further, information input devices need not be hard-wired to medical instruments, information may be transferred through a wireless connection as well. Additionally, other types of modules may be connected to the pump modules or to the control unit such as a syringe pump module, patient controlled analgesic module, End Tidal CO₂ monitoring module, oximeter monitoring module, or the like.

In some embodiments, the pressure measurements from the upstream and/or downstream pressure sensors are transmitted to a server or other coordination device, and the methods disclosed herein are implemented on the server or other coordination device. For example, a pressure sensor may be used to determine a pressure or force within the infusion line downstream of the pump (e.g., between the patient and the pump). More sophisticated and computationally intensive approaches like machine-learning can be implemented on a server (or on a control unit with a larger memory and/or CPU resources). In some embodiments, machine learning is used to identify empty conditions based on pressure signals received from the pump.

FIG. 1B depicts a second example of a patient care unit 10 with connected functional modules, according to aspects of the subject technology. The patient care unit (or "PCU") may include various ancillary medical devices such as an infusion pump, a vital signs monitor, a medication dispensing device (e.g., cabinet, tote), a medication preparation device, an automated dispensing device, a module coupled with one of the aforementioned (e.g., a syringe pump module configured to attach to an infusion pump), or other similar devices.

In the depicted example, patient care unit 12 comprises a control module 14, also referred to as main frame infusion controller 14, connected to one or more functional modules 16, 18, 20, 22. Main frame infusion controller 14 includes a central processing unit (CPU) 50 connected to a memory, for example, random access memory (RAM) 58, and one or more interface devices such as user interface device 54, a coded data input device 60, a network connection 52, and an auxiliary interface 62 for communicating with additional modules or devices. Main frame infusion controller 14 also, although not necessarily, includes a main non-volatile storage unit 56, such as a hard disk drive or non-volatile flash memory, for storing software and data and one or more internal buses 64 for interconnecting the aforementioned elements. Patient care unit 12 may include or incorporate pumps, physiological monitors (e.g., heart rate, blood pressure, ECG, EEG, pulse oximeter, and other patient monitors), therapy devices, and other drug delivery devices may be utilized according to the teachings set forth herein.

In various implementations, user interface device 54 is a touch screen for displaying information to a user and allowing a user to input information by touching defined areas of the screen. Additionally, or in the alternative, user interface device 54 could include any means for displaying and inputting information, such as a monitor, a printer, a keyboard, softkeys, a mouse, a track ball and/or a light pen. Data input device 60 may be a bar code reader capable of scanning and interpreting data printed in bar coded format. Additionally or in the alternative, data input device 60 can be any device for entering coded data into a computer, such as a device(s) for reading a magnetic strips, wireless communication (e.g., radio frequency or Bluetooth), radio-frequency identification (RFID) devices whereby digital data encoded in RFID tags or smart labels (defined below) are captured by the reader 60 via radio waves, PCMCIA smart cards, radio frequency cards, memory sticks, CDs, DVDs, or any other analog or digital storage media. Other examples of data input device 60 include a voice activation or recognition device or a portable personal data assistant (PDA). Depending upon the types of interface devices used, user interface device 54 and data input device 60 may be the same device. Although data input device 60 is shown as being disposed within main frame infusion controller 14, it is recognized that data input device 60 may be integral within pharmacy system or located externally and communicating with pharmacy system through an RS-232 serial interface or any other appropriate communication means. Auxiliary interface 62 may be an RS-232 communications interface, however any other means for communicating with a peripheral device such as a printer, patient monitor, infusion pump or other medical device may be used without departing from the subject technology. Additionally, data input device 60 may be a separate functional module, such as modules 16, 18, 20 and 22, and configured to communicate with a main frame infusion (controller) device 14, or any other system on the network, using suitable programming and communication protocols.

Network connection 52 may be a wired or wireless connection, such as by Ethernet, WiFi, BLUETOOTH, an integrated services digital network (ISDN) connection, a digital subscriber line (DSL) modem or a cable modem. Any direct or indirect network connection may be used, including, but not limited to a telephone modem, an MIB system, an RS232 interface, an auxiliary interface, an optical link, an infrared link, a radio frequency link, a microwave link or a WLANS connection or other wireless connection.

Functional modules 16, 18, 20, 22 are any devices for providing care to a patient or for monitoring patient condition. At least one of functional modules 16, 18, 20, 22 may be an infusion pump module such as an intravenous infusion pump for delivering medication or other fluid to a patient. For the purposes of this discussion, functional module 16 is an infusion pump module. Each of functional modules 16, 18, 20, 22 may be any patient treatment or monitoring device including, but not limited to, an infusion pump, a syringe pump, a PCA pump, an epidural pump, an enteral pump, a blood pressure monitor, a pulse oximeter, an EKG monitor, an EEG monitor, a heart rate monitor, an intracranial pressure monitor, or the like. Functional module 16, 18, 20 and/or 22 may be a printer, scanner, bar code reader, near-field communication reader, RFID reader, radio frequency (RF) transmission, BLUETOOTH, or any other peripheral input, output or input/output device.

Data to and from the various data sources can be converted into network-compatible data with existing technology, and movement of the information between the medical device and network can be accomplished by a variety of means. For example, patient care unit 12 and network 10 may communicate via automated interaction, manual interaction or a combination of both automated and manual interaction. Automated interaction may be continuous or intermittent and may occur through direct network connection 52 (as shown in FIG. 1A), or through RS232 links, MIB systems, RF links such as BLUETOOTH, IR links, WLANS, digital cable systems, telephone modems or other wired or wireless communication means. Manual interaction between patient care unit 10 and a network may involve physically transferring, intermittently or periodically, data between systems using, for example, user interface device 54, coded data input device 60, bar codes, computer disks, portable data assistants, memory cards, or any other media for storing data.

FIGS. 2A through 2G depict an example intelligent lockbox 200 for securing a patient care unit 10, according to various aspects of the subject technology. FIG. 2A depicts a perspective view of the example lockbox 200 enclosing the patient care unit. According to various implementations, the lockbox 200 includes a housing that completely encompasses the patient care unit 10, including any attached modules 16, 18, 20, 22 and associated medication, and secures the unit and modules from physical manipulation by a person who does not have access to the lockbox. **In** the depicted example, one module encompassed and secured by lockbox 200 includes syringe pump 30. In this example, the medication within the syringe pump is secured by way of being enclosed within the lockbox. In some implementations, the lockbox may be extended to house and secure a medication bag for use with different types of pumps (e.g., peristaltic pumps).

A rear side of the housing of lockbox 200 (see FIGS. 2B, 2E, 2F) is positioned between the patient care unit 10 and a pole 2 or other structure to which the patient care unit is mounted. As will be described further, the rear side of the lockbox is configured to form around and secure to a mounting structure. The lockbox includes at least one access door 202. In the depicted example, the lockbox 200 includes left and right bifold access doors 202a and 202b that, as will be described further, open to the side to reveal a front face of the patient control unit 10.

FIG. 2B depicts a perspective view of an unlocking and opening of the lockbox of FIG. 2A, according to various aspects of the subject technology. According to various implementations, the housing of lockbox 200 includes an electronically controlled lock and a series of sliding doors that may be actuated by a corresponding series of motors and mechanical gears (not shown) to move the doors upon the lockbox 200 being opened. In the depicted example, the doors include a retractable multi-folding door 202 on each side of the housing that, when closed, form a front face of the lockbox housing, and a top face 204 (or lid) that closes to form a top of the lockbox housing. When closed, the top face 204 is integrally coupled with the front face of the housing, made up by the doors 202a, 202b.

As shown, when the lockbox 200 receives an authorized signal to trigger opening of the lockbox, the motors and gears operate to retract the multi-folding doors in opposing left and right directions 206a, 206b to expose the respective portions of the front face of the patient care unit 10 within the housing. Each door is physically and hingedly coupled to a respective left or right side wall 208a, 208b of the lockbox housing. Contemporaneous with the retraction of the multi-folding doors, the walls 208a, 208b are moved backwards in a direction 210 to expose and provide access to at least a portion of the sides of the patient care unit 10. Contemporaneous with the retraction of the doors and sides, the top face 204 may swing open according to an upward directional arc 212 to reveal and provide access to at least a portion of the top of the patient care unit 10. In some implementations not covered by the claims, the lockbox may open without motors. In such implementations, the gears may be interlinked so that, when the door and/or top of the lockbox is manually opened (e.g., after a user is granted access), the doors, top, and or sides move collectively together to open the lockbox.

According to various implementations, the lockbox 200 includes a lockbox control interface device 214 that is integral with the housing (e.g., embedded within the housing or securely attached to the housing). In the depicted example, control interface device 214 is embedded in a door. The control interface device 214 may be electronically or communicatively coupled to the various motors and gears within the lockbox housing and configured to operate the motors to control opening and closing of the various doors, sides, and lid of the housing.

In some implementations, the control interface 214 is configured to receive a user credential and/or other user input. In some implementations, control interface 214 may include a touch-screen for receiving the input. Additionally or in the alternative, control interface 214 may include a traditional keypad. In some implementations, the lockbox control interface 214 includes a wireless communication module (e.g., radio-frequency identification (RFID)) scanner to receive the credential from a (e.g., RFID or BLUETOOTH) transmitting device. As will be described further, the control interface 214 may be communicatively connected to the control unit 14 or other processing device of the patient control unit 10 to authenticate/validate credentials received via the interface 214 or to receive a signal from the patient care device 10 to open the lockbox 200. Upon receiving an authorized credential, the control interface 214 may automatically retract the doors 202, lid 204, and/or sides 208 to provide user access to the patient care device. In some implementations, on receiving the authorized credential, the control interface 214 may provide (e.g., via the touch screen display) an extension of the various displays 4 and inputs 6 of the patient care device 10 (see FIG. 1A).

FIG. 2C depicts a front view of the lockbox in a closed and secure state, according to various aspects of the subject technology. FIG. 2D depicts a font view of the lockbox in an open and unsecured state, according to various aspects of the subject technology. FIG. 2E depicts a side view of the lockbox in a closed and secure state, according to various aspects of the subject technology. FIG. 2F depicts a side view of the lockbox in an open and unsecured state, according to various aspects of the subject technology. In the depicted example, the patient care device 10 is mounted to pole 2 by a horizontal mount 4. It is understood that other known mounting hardware may be utilized to secure the patient care device 10 to the pole 2 or other structure.

FIG. 2G depicts an exploded view of the lockbox 200, according to various aspects of the subject technology. The rear face 216 of the lockbox housing includes a elongated horizontal channel or slot 218 that, when a side 220a of the housing is removed, is open on one end. In this regard, the slot 218 may be positioned at the same height within the face 216 relative to the mount 4 on the patient care device 10, so that the slot 218 may be guided over the mount 4 to secure the lockbox to the patient care device 10. Accordingly, the lockbox can be secured by the rear face 216 being locked in place around the mount 4 between the patient care device 10 and the pole 2 (or other mounting surface). The only way to remove the lockbox from the patient care device 10 is to access the lockbox and to disconnect and remove the side 220a from the rear face 216 as shown.

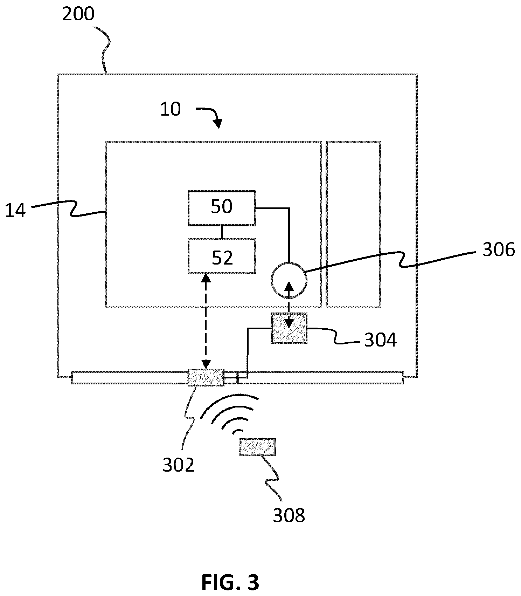

FIG. 3 depicts a system for securing an infusion device by authenticating a user to the infusion device via an intelligent lockbox, according to various aspects of the subject technology. In the depicted example, a lockbox 200 securably encloses a patient care unit 10, as previously described. The lockbox 200 includes a control interface 302 configured to receive user credentials. According to various implementations, the control interface 302 includes wireless circuitry and processing that is configured to be paired with a communication interface 52 of the PCU 10, for example, prior to the lockbox being secured around the PCU 10. In some implementations, the communication between the lockbox control interface 302 and the communication interface 52 of the PCU 10 may be in the form of a wired connection (within the lockbox).

According to various implementations, the lockbox is self powered, and includes a power supply 304 within the lockbox (e.g., inaccessible to a person outside the lockbox when the lockbox is secured). In some implementations, power for the lockbox doors and/or control interface 302 may be provided or supplemented by the infusion device (e.g., PCU 10). In such implementations, lockbox power interface 304 may include a wired connection configured to connect to and/or plug into a power charger 306 (e.g., outlet) provided by the infusion device 10 (e.g., on the main frame control unit 14). In some implementations, the lockbox 200 may be powered with PoE (power over ethernet). In some implementations, the lockbox power interface 304 may include wireless charging capabilities, and may be positioned adjacent a wireless power charger 306 attached to or integrated in the infusion device such as to receive charging and/or power from the infusion device when the lockbox is secured to the infusion device. In FIG. 3, supply 304 is positioned on the bottom internal side of the lockbox 200, adjacent to power charger 306. In some implementations, power supply 304 may be positioned directly below charger 306 for efficient wireless charging.

In some implementations, the lockbox control interface 302 includes an input device such as a touch screen device. In this regard, the touchscreen device may display a lockbox user interface that prompts for and/or receives credentials. For example, the touchscreen may prompt for a username and password and then provide a virtual keypad for entry of the credentials. In some implementations, the interface 302 may include a biometric scanner such as a fingerprint reader. In some implementations, the lockbox control interface 302 includes a wireless scanner (e.g., RFID or BLUETOOTH receiver) configured to receive the user credential wirelessly from a transmitting device 308 associated with a user. For example, a user may scan his or her badge 308 at the interface 302 to provide a credential stored within an RFID device within the badge to the interface 302.

Accordingly, the lockbox interface 302 facilitates transmission of a authentication/authorization credential to the infusion device for authorization to the lockbox, for example, by receiving the credential wirelessly from a transmitting device 308 and retransmitting it (or a representation of it) to a control processor 50 within the infusion device 10. The processor 50 may then authenticate the user associated with the credential and, upon authentication, the processor may authorize the user to the lockbox. The processor 50 may signal the lockbox interface to indicate that the user is authorized to perform an action with regard to the lockbox. As will be described further, the action may include causing the lockbox to open (as shown in FIG. 2) or activate the lockbox user interface 302 to function as an extension of the control unit 14 interface (e.g., display 4 and/or control keys 6).

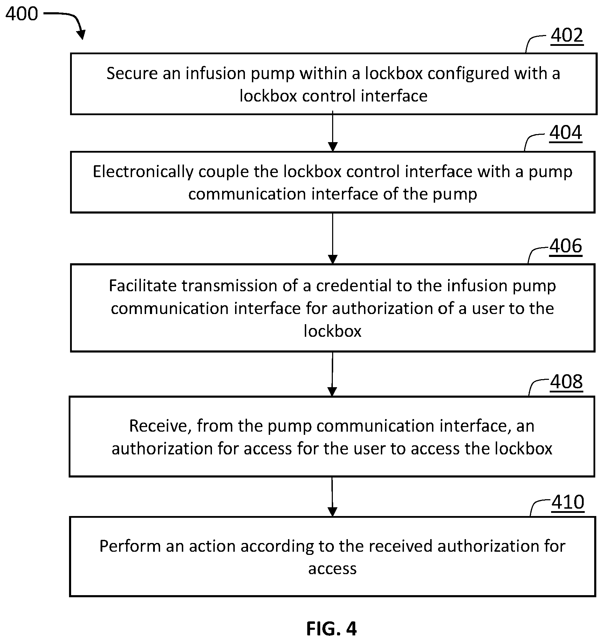

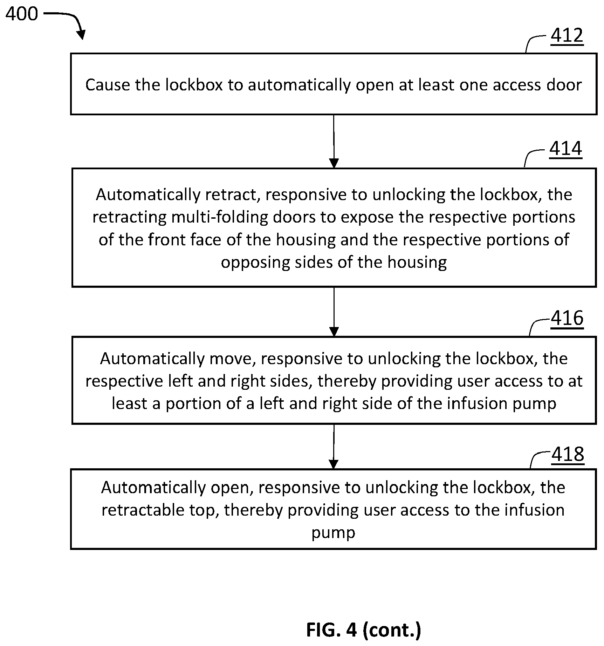

FIG. 4 depicts an example process 400 for securing an infusion device using an intelligent lockbox, according to aspects of the subject technology. For explanatory purposes, the various blocks of example process 400 are described herein with reference to FIGS. 1-3, and the associated components and/or processes described herein. The one or more of the blocks of process 400 may be implemented, for example, by one or more computing devices including, for example, infusion control module 14, PCU 10, or one or more of modules 16, 18, 20, 22, and 30. In some implementations, one or more of the blocks may be implemented based on one or more machine learning algorithms. In some implementations, one or more of the blocks may be implemented apart from other blocks, and by one or more different processors or devices. Further, for explanatory purposes, to the extent that the blocks of example process 400 are described as occurring in serial, or linearly, in some implementations, multiple blocks of example process 400 may occur in parallel. In addition, the blocks of example process 400 need not be performed in the order shown and/or one or more of the blocks of example process 400 need not be performed.

In the depicted process 400, an infusion device (e.g., a pump) is secured within a lockbox (402). According to various implementations, the lockbox is configured to enclose and prevent access to at least a control interface of the infusion pump and a medication receptacle of the infusion pump (e.g., an infusion bag). As described with regard to FIG. 3, the lockbox includes a lockbox control interface 302 configured to obtain authorization for access to the lockbox from a pump communication interface 52 of the infusion pump. In some implementations, the lockbox control interface includes a scanner configured to receive the user credential wirelessly from a transmitting device (e.g., a RFID or BLUETOOTH transmitter) associated with a user.

In some implementations, securing the infusion device may include locking the lockbox doors 202 and/or activating the control interface 302 to require authorized credentials to unlock the doors.

The lockbox control interface 302 is electronically coupled with the pump communication interface 52 (404). In some implementations, electronically coupling the lockbox control interface with the pump communication interface includes wirelessly pairing the lockbox control interface with the pump communication interface, for example, using a BLUETOOTH connection. In some implementation, the coupling includes a wired connection within the lockbox 200 (secured from outside tampering).

The lockbox 200 facilitates transmission of a credential to the infusion pump communication interface for authorization to the lockbox. Accordingly, in some implementations, a credential received by the lockbox control interface 302 and transmitted to the infusion pump communication interface 52 for authorization of the user to the lockbox (406). In some implementations, the credential may be entered via a lockbox user interface displayed or provided at the control interface 302. In some implementations, the credential is received from a badge scanned by the RFID scanner at the control interface 302. In some implementations, facilitating transmission by the lockbox includes the RFID credential being scanned by the pump communication interface 52 through a housing of the lockbox.

The process 500 continues with the lockbox control interface 302 receiving, from the pump communication interface 52, an authorization for access for the user to access the lockbox (408). Accordingly, the authorization for access may be received by the lockbox control interface 302 from the pump communication interface 52 responsive to transmitting the user credential to the pump communication interface 52 (and the pump authorizing the credential).

The lockbox control interface 302 performs an action according to the received authorization for access (410). According to some implementations, performing the action includes automatically (without further user intervention) unlocking the lockbox to allow user access to a user interface of the infusion pump and the medication receptacle. In some implementations, performing the action includes activating the pump user interface to receive user input while the lockbox remains locked. For example, contemporaneous with opening the lockbox, the pump's display(s) may switch from an off state (e.g., power off and/or unable to receive input) to an activated state (e.g., power and lights switch on and ready to receive input). In this regard, the lockbox user interface may extend (e.g., virtually) the pump's user interface such as the display 4 or control keys 6. The authorized user may then provide input for controlling the pump via the lockbox user interface 302 while the lockbox remains locked. The lockbox control interface 302 receives the user input via the activated lockbox user interface, and transmits the user input to the pump communication interface 52 (and processor 50) for operation for the infusion pump in accordance with the authorization for access (e.g., as if the input had been received directly by the pump user interface).

FIG. 4 continues with an optional sub-process for the action performed according to the authorization access, according to aspects of the subject technology. According to various implementations, as described previously, the lockbox includes retracting multi-folding doors and a series of motors and gears (not shown) to move and/or retract and/or lock or unlock the doors.

According to the depicted examples, responsive to unlocking the lockbox the lockbox control interface causes the lockbox to automatically open at least one access door to expose the user interface or the medication receptacle (412). As described previously, the lockbox includes a housing enclosing the patient care unit and/or infusion pump 10. The retractable multi-folding doors are configured to form the front face of the housing when the lockbox is closed. The multi-folding doors may include a left bifold door 202a and a right bifold door 202b that open like folding closet doors, with each half of the bifold doors folding over the other as the door opens. In this regard, the process continues with the lockbox (by way of motors and/or gears) automatically retracting, responsive to unlocking the lockbox, the retracting multi-folding doors to expose the respective portions of the front face of the housing and the respective portions of opposing sides of the housing, thereby providing user access to the infusion pump (414).

According to some implementations, once the doors 202a, 202b are completely open, the respective left and right sides of the lockbox 200 are configured to move in a direction 210 away from the front face of the housing when the lockbox is unlocked. In this regard, the process of unlocking the lockbox further includes automatically moving, responsive to unlocking the lockbox, the respective left and right sides, thereby providing user access to at least a portion of a left and right side of the infusion pump (416).

As shown in FIGS. 2B and 2D, the lockbox 200 further may further include a retractable top that, when closed, is integral to the front face. In this regard, the process of unlocking the lockbox further includes automatically opening, responsive to unlocking the lockbox, the retractable top, thereby providing user access to the infusion pump (418). In some implementations, the lockbox 200 communicates opening and closing actions to the infusion pump, and may receive information from the pump regarding interactions with the pump by the user. In some implementations, the lockbox 200 may automatically close after a predetermined period of time (e.g., after two minutes of being open with no interaction with the pump).

Many of the above-described example process 400 and related features and applications, may also be implemented as software processes that are specified as a set of instructions recorded on a computer readable storage medium (also referred to as computer readable medium), and may be executed automatically (e.g., without user intervention). When these instructions are executed by one or more processing unit(s) (e.g., one or more processors, cores of processors, or other processing units), they cause the processing unit(s) to perform the actions indicated in the instructions. Examples of computer readable media include, but are not limited to, CD-ROMs, flash drives, RAM chips, hard drives, EPROMs, etc. The computer readable media does not include carrier waves and electronic signals passing wirelessly or over wired connections.

The term "software" is meant to include, where appropriate, firmware residing in read-only memory or applications stored in magnetic storage, which can be read into memory for processing by a processor. Also, in some implementations, multiple software aspects of the subject disclosure can be implemented as sub-parts of a larger program while remaining distinct software aspects of the subject disclosure. In some implementations, multiple software aspects can also be implemented as separate programs. Finally, any combination of separate programs that together implement a software aspect described here is within the scope of the subject disclosure. In some implementations, the software programs, when installed to operate on one or more electronic systems, define one or more specific machine implementations that execute and perform the operations of the software programs.

A computer program (also known as a program, software, software application, script, or code) can be written in any form of programming language, including compiled or interpreted languages, declarative or procedural languages, and it can be deployed in any form, including as a stand-alone program or as a module, component, subroutine, object, or other unit suitable for use in a computing environment. A computer program may, but need not, correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

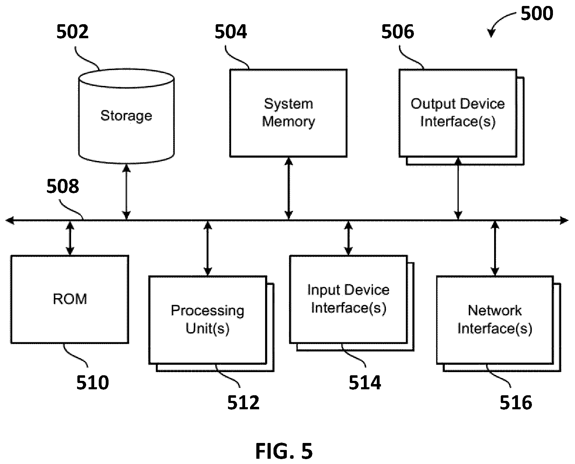

FIG. 5 is a conceptual diagram illustrating an example electronic system 500 for securing an infusion device using an intelligent lockbox, according to aspects of the subject technology. Electronic system 500 may be a computing device for execution of software associated with one or more portions or steps of process 500, or components and methods provided by FIGS. 1-4, including but not limited to computing hardware within the PCU 10, main frame infusion controller 14, control module 14, functional modules 16, 18, 20, 22, 30, and/or any computing devices or associated modules or terminals disclosed herein. Electronic system 500 may be a personal computer or a mobile device such as a smartphone, tablet computer, laptop, PDA, an augmented reality device, a wearable such as a watch or band or glasses, or combination thereof, or other touch screen or television with one or more processors embedded therein or coupled thereto, or any other sort of computer-related electronic device having network connectivity.

Electronic system 500 may include various types of computer readable media and interfaces for various other types of computer readable media. In the depicted example, electronic system 500 includes a bus 508, processing unit(s) 512, a system memory 504, a read-only memory (ROM) 510, a permanent storage device 502, an input device interface 514, an output device interface 506, and one or more network interfaces 516. In some implementations, electronic system 500 may include or be integrated with other computing devices or circuitry for operation of the various components and methods previously described.

Bus 508 collectively represents all system, peripheral, and chipset buses that communicatively connect the numerous internal devices of electronic system 500. For instance, bus 508 communicatively connects processing unit(s) 512 with ROM 510, system memory 504, and permanent storage device 502.

From these various memory units, processing unit(s) 512 retrieves instructions to execute and data to process, in order to execute the processes of the subject disclosure. The processing unit(s) can be a single processor or a multi-core processor in different implementations.

ROM 510 stores static data and instructions that are needed by processing unit(s) 512 and other modules of the electronic system. Permanent storage device 502, on the other hand, is a read-and-write memory device. This device is a non-volatile memory unit that stores instructions and data even when electronic system 500 is off. Some implementations of the subject disclosure use a mass-storage device (such as a magnetic or optical disk and its corresponding disk drive) as permanent storage device 502.

Other implementations use a removable storage device (such as a floppy disk, flash drive, and its corresponding disk drive) as permanent storage device 502. Like permanent storage device 502, system memory 504 is a read-and-write memory device. However, unlike storage device 502, system memory 504 is a volatile read-and-write memory, such as, random access memory. System memory 504 stores some of the instructions and data that the processor needs at runtime. In some implementations, the processes of the subject disclosure are stored in system memory 504, permanent storage device 502, and/or ROM 510. From these various memory units, processing unit(s) 512 retrieves instructions to execute and data to process in order to execute the processes of some implementations.

Bus 508 also connects to input and output device interfaces 514 and 506. Input device interface 514 enables the user to communicate information and select commands to the electronic system. Input devices used with input device interface 514 include, e.g., alphanumeric keyboards and pointing devices (also called "cursor control devices"). Output device interfaces 506 enables, e.g., the display of images generated by the electronic system 500. Output devices used with output device interface 506 include, e.g., printers and display devices, such as cathode ray tubes (CRT) or liquid crystal displays (LCD). Some implementations include devices such as a touchscreen that functions as both input and output devices.

Also, as shown in FIG. 5, bus 508 also couples electronic system 500 to a network (not shown) through network interfaces 516. Network interfaces 516 may include, e.g., a wireless access point (e.g., Bluetooth or WiFi) or radio circuitry for connecting to a wireless access point. Network interfaces 516 may also include hardware (e.g., Ethernet hardware) for connecting the computer to a part of a network of computers such as a local area network ("LAN"), a wide area network ("WAN"), wireless LAN, or an Intranet, or a network of networks, such as the Internet. Any or all components of electronic system 500 can be used in conjunction with the subject disclosure.

These functions described above can be implemented in computer software, firmware, or hardware. The techniques can be implemented using one or more computer program products. Programmable processors and computers can be included in or packaged as mobile devices. The processes and logic flows can be performed by one or more programmable processors and by one or more programmable logic circuitry. General and special purpose computing devices and storage devices can be interconnected through communication networks.

Some implementations include electronic components, such as microprocessors, storage and memory that store computer program instructions in a machine-readable or computer-readable medium (also referred to as computer-readable storage media, machine-readable media, or machine-readable storage media). Some examples of such computer-readable media include RAM, ROM, read-only compact discs (CD-ROM), recordable compact discs (CD-R), rewritable compact discs (CD-RW), read-only digital versatile discs (e.g., DVD-ROM, dual-layer DVD-ROM), a variety of recordable/rewritable DVDs (e.g., DVD-RAM, DVD-RW, DVD+RW, etc.), flash memory (e.g., SD cards, mini-SD cards, micro-SD cards, etc.), magnetic and/or solid state hard drives, read-only and recordable Blu-Ray^{®} discs, ultra density optical discs, any other optical or magnetic media, and floppy disks. The computer-readable media can store a computer program that is executable by at least one processing unit and includes sets of instructions for performing various operations. Examples of computer programs or computer code include machine code, such as is produced by a compiler, and files including higher-level code that are executed by a computer, an electronic component, or a microprocessor using an interpreter.

While the above discussion primarily refers to microprocessor or multi-core processors that execute software, some implementations are performed by one or more integrated circuits, such as application specific integrated circuits (ASICs) or field programmable gate arrays (FPGAs). In some implementations, such integrated circuits execute instructions that are stored on the circuit itself.

As used in this specification and any claims of this application, the terms "computer", "server", "processor", and "memory" all refer to electronic or other technological devices. These terms exclude people or groups of people. For the purposes of the specification, the terms display or displaying means displaying on an electronic device. As used in this specification and any claims of this application, the terms "computer readable medium" and "computer readable media" are entirely restricted to tangible, physical objects that store information in a form that is readable by a computer. These terms exclude any wireless signals, wired download signals, and any other ephemeral signals.

To provide for interaction with a user, implementations of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; e.g., feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; e.g., by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), an internetwork (e.g., the Internet), and peer-to-peer networks (e.g., ad hoc peer-to-peer networks).

The computing system can include clients and servers. A client and server are generally remote from each other and may interact through a communication network. The relationship of client and server arises by virtue of computer programs running on the respective computers and having a client-server relationship to each other. In some embodiments, a server transmits data (e.g., an HTML page) to a client device (e.g., for purposes of displaying data to and receiving user input from a user interacting with the client device). Data generated at the client device (e.g., a result of the user interaction) can be received from the client device at the server.

Those of skill in the art would appreciate that the various illustrative blocks, modules, elements, components, methods, and algorithms described herein may be implemented as electronic hardware, computer software, or combinations of both. To illustrate this interchangeability of hardware and software, various illustrative blocks, modules, elements, components, methods, and algorithms have been described above generally in terms of their functionality. Whether such functionality is implemented as hardware or software depends upon the particular application and design constraints imposed on the overall system. The described functionality may be implemented in varying ways for each particular application. Various components and blocks may be arranged differently (e.g., arranged in a different order, or partitioned in a different way) all without departing from the scope of the subject technology.

It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

Further Consideration:
It is understood that the specific order or hierarchy of steps in the processes disclosed is an illustration of example approaches. Based upon design preferences, it is understood that the specific order or hierarchy of steps in the processes may be rearranged. Some of the steps may be performed simultaneously. The accompanying method claims present elements of the various steps in a sample order, and are not meant to be limited to the specific order or hierarchy presented.

The previous description is provided to enable any person skilled in the art to practice the various aspects described herein. The previous description provides various examples of the subject technology, and the subject technology is not limited to these examples. Various modifications to these aspects will be readily apparent to those skilled in the art, and the generic principles defined herein may be applied to other aspects. Thus, the claims are not intended to be limited to the aspects shown herein, but is to be accorded the full scope consistent with the language claims, wherein reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention described herein.

The predicate words "configured to", "operable to", and "programmed to" do not imply any particular tangible or intangible modification of a subject, but, rather, are intended to be used interchangeably. For example, a processor configured to monitor and control an operation, or a component may also mean the processor being programmed to monitor and control the operation or the processor being operable to monitor and control the operation. Likewise, a processor configured to execute code can be construed as a processor programmed to execute code or operable to execute code.

The term automatic, as used herein, may include performance by a computer or machine without user intervention; for example, by instructions responsive to a predicate action by the computer or machine or other initiation mechanism. The word "example" is used herein to mean "serving as an example or illustration." Any aspect or design described herein as "example" is not necessarily to be construed as preferred or advantageous over other aspects or designs.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such as an "embodiment" may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such as a "configuration" may refer to one or more configurations and vice versa.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

As used herein, the terms "determine" or "determining" encompass a wide variety of actions. For example, "determining" may include calculating, computing, processing, deriving, generating, obtaining, looking up (e.g., looking up in a table, a database or another data structure), ascertaining and the like via a hardware element without user intervention. Also, "determining" may include receiving (e.g., receiving information), accessing (e.g., accessing data in a memory) and the like via a hardware element without user intervention. "Determining" may include resolving, selecting, choosing, establishing, and the like via a hardware element without user intervention.

As used herein, the terms "provide" or "providing" encompass a wide variety of actions. For example, "providing" may include storing a value in a location of a storage device for subsequent retrieval, transmitting a value directly to the recipient via at least one wired or wireless communication medium, transmitting or storing a reference to a value, and the like. "Providing" may also include encoding, decoding, encrypting, decrypting, validating, verifying, and the like via a hardware element.

As used herein, the term "message" encompasses a wide variety of formats for communicating (e.g., transmitting or receiving) information. A message may include a machine readable aggregation of information such as an XML document, fixed field message, comma separated message, JSON, a custom protocol, or the like. A message may, in some implementations, include a signal utilized to transmit one or more representations of the information. While recited in the singular, it will be understood that a message may be composed, transmitted, stored, received, etc. in multiple parts.

As used herein, the term "selectively" or "selective" may encompass a wide variety of actions. For example, a "selective" process may include determining one option from multiple options. A "selective" process may include one or more of: dynamically determined inputs, preconfigured inputs, or user-initiated inputs for making the determination. **In** some implementations, an n-input switch may be included to provide selective functionality where n is the number of inputs used to make the selection.

As user herein, the terms "correspond" or "corresponding" encompasses a structural, functional, quantitative and/or qualitative correlation or relationship between two or more objects, data sets, information and/or the like, preferably where the correspondence or relationship may be used to translate one or more of the two or more objects, data sets, information and/or the like so to appear to be the same or equal. Correspondence may be assessed using one or more of a threshold, a value range, fuzzy logic, pattern matching, a machine learning assessment model, or combinations thereof.

In any embodiment, data generated or detected can be forwarded to a "remote" device or location, where "remote," means a location or device other than the location or device at which the program is executed. For example, a remote location could be another location (e.g., office, lab, etc.) in the same city, another location in a different city, another location in a different state, another location in a different country, etc. As such, when one item is indicated as being "remote" from another, what is meant is that the two items can be in the same room but separated, or at least in different rooms or different buildings, and can be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. Examples of communicating media include radio or infra-red transmission channels as well as a network connection to another computer or networked device, and the internet or including email transmissions and information recorded on websites and the like.

## Claims

1. A system (500) for securing a pump, comprising:
a lockbox (200) comprising a housing enclosing an infusion pump (30), the housing comprising a retractable multi-folding door (202a, 202b) on each side of a front face of the housing that, when closed, form a front face of the housing, and a retractable top (204) that, when closed, is integrally coupled to the front face; and
a lockbox control interface (214) associated with the lockbox configured to obtain authorization for access to the lockbox from a pump communication interface (52) of the infusion pump and enclosed within the lockbox, the lockbox control interface comprising a processor (50) configured to:
electronically couple the lockbox control interface with the pump communication interface;
facilitate transmission of a wireless credential to the pump communication interface for authorization to the lockbox;
receive, from the pump communication interface, the authorization for access based on the wireless credential; and
causing, according to the received authorization for access, unlocking of the lockbox to allow user access to a user interface (4, 6) of the infusion pump and a medication receptacle of the infusion pump by automatically retracting the multi-folding doors to expose at least a portion of a front face of the infusion pump and automatically opening the retractable top, thereby providing user access to at least a portion of a top surface of the infusion pump.

2. The system of Claim 1, wherein the processor is further configured to:
receive a user credential via the lockbox control interface; and
transmit, via the lockbox control interface, the received user credential to the pump communication interface, wherein the authorization for access is received by the lockbox control interface from the pump communication interface responsive to transmitting the user credential to the pump communication interface.

3. The system of Claim 2, wherein the lockbox control interface comprises a scanner configured to receive the user credential wirelessly from a transmitting device associated with a user, the processor being further configured to:
receive the user credential from a badge scanned by the scanner.

4. The system of any one of Claims 1-3, wherein electronically coupling the lockbox control interface with the pump communication interface comprises:
wirelessly pairing the lockbox control interface with the pump communication interface.

5. The system of Claim 1, wherein the processor is further configured to:
automatically switch, responsive to unlocking the lockbox, the user interface of the infusion pump from an off state to an activated state.

6. The system of Claim 1, wherein the lockbox further comprises respective left and right sides (208a, 208b) configured to move in a direction away from the front face of the housing when the lockbox is unlocked, wherein the processor is further configured to:
automatically move, responsive to unlocking the lockbox, the respective left and right sides, thereby providing user access to at least a portion of a left and right side of the infusion pump.

7. The system of any one of Claims 1-6, wherein the lockbox control interface is configured to receive user input, wherein the processor is further configured to:
activate the lockbox control interface to receive user input when the lockbox is unlocked;
receive the user input via the activated lockbox control interface; and
transmit, after the authorization for access, the user input to the pump communication interface for operation of the infusion pump in accordance with the authorization for access.

8. The system of any one of Claims 1, wherein the pump communication interface comprises a scanner and is configured to receive a user credential wirelessly via the scanner from a transmitting device associated with a user, and to transmit the authorization for access based on the wireless credential to the lockbox control interface.

9. A method (400) for securing a pump, comprising:
securing (402) at least a portion of an infusion pump within a lockbox, the lockbox comprising a housing enclosing an infusion pump, the housing comprising a retractable multi-folding door on each side of a front face of the housing that, when closed, form a front face of the housing, and a retractable top that, when closed, is integrally coupled to the front face, the lockbox comprising a lockbox control interface configured to obtain authorization for access to the lockbox from a pump communication interface of the infusion pump and enclosed within the lockbox;
electronically coupling (404) the lockbox control interface with the pump communication interface;
facilitating (406) transmission of a wireless credential to the pump communication interface for authorization to the lockbox;
receiving (408), by the lockbox control interface from the pump communication interface, the authorization for access based on the wireless credential; and
causing, by the lockbox control interface, according to the received authorization for access, an unlocking of the lockbox to allow user access to a user interface of the infusion pump and a medication receptacle of the infusion pump by automatically retracting (414) the multi-folding doors to expose at least a portion of a front face of the infusion pump and automatically opening (418) the retractable top, thereby providing user access to at least a portion of a top surface of the infusion pump.

10. The method of Claim 9, further comprising:
receiving a user credential via the lockbox control interface; and
transmitting, by the lockbox control interface, the received user credential to the pump communication interface, wherein the authorization for access is received by the lockbox control interface from the pump communication interface responsive to transmitting the user credential to the pump communication interface.

11. The method of Claim 9, wherein the pump communication interface comprises a scanner configured to receive a user credential through the lockbox wirelessly from a transmitting device associated with a user outside the lockbox, wherein the authorization for access is received by the lockbox control interface from the pump communication interface responsive to the user credential being scanned at the pump communication interface.

12. The method of any one of Claims 9-11, wherein electronically coupling the lockbox control interface with the pump communication interface comprises:
wirelessly pairing the lockbox control interface with the pump communication interface.

13. The method of Claim 9, the method further comprising:
automatically switching, responsive to unlocking the lockbox, the user interface of the infusion pump from an off state to an activated state.

14. The method of Claim 9, wherein the lockbox further comprises respective left and right sides configured to move in a direction away from the front face of the housing when the lockbox is unlocked, the method further comprising:
automatically moving, responsive to unlocking the lockbox, the respective left and right sides, thereby providing user access to at least a portion of a left and right side of the infusion pump.

15. The method of any one of Claims 9-14, wherein the method further comprises:
activating the lockbox control interface to receive user input when the lockbox is unlocked;
receiving user input via the activated lockbox control interface; and
transmitting, after the authorization for access, the user input to the pump communication interface for operation of the infusion pump in accordance with the authorization for access.

## Patentansprüche

1. System (500) zum Sichern einer Pumpe, umfassend:
eine Lockbox (200) umfassend ein Gehäuse, das eine Infusionspumpe (30) einschließt, wobei das Gehäuse eine einziehbare, mehrfach faltbare Tür (202a, 202b) an jeder Seite einer Vorderfläche des Gehäuses umfasst, die, wenn sie geschlossen sind, eine Vorderfläche des Gehäuses bilden, und eine einziehbare Oberseite (204), die, wenn sie geschlossen ist, integral mit der Vorderfläche gekoppelt ist; und
eine mit der Lockbox verbundene und innerhalb der Lockbox eingeschlossene Lockbox-Steuerschnittstelle (214), die dazu ausgebildet ist, eine Berechtigung für den Zugriff auf die Lockbox von einer Pumpenkommunikationsschnittstelle (52) der Infusionspumpe zu erhalten, wobei die Lockbox-Steuerschnittstelle einen Prozessor (50) umfasst, der dazu ausgebildet ist:
die Lockbox-Steuerschnittstelle elektronisch mit der Pumpenkommunikationsschnittstelle zu koppeln;
die Übertragung eines drahtlosen Berechtigungsnachweises an die Pumpenkommunikationsschnittstelle zur Berechtigung für die Lockbox zu ermöglichen;
von der Pumpenkommunikationsschnittstelle die Berechtigung für den Zugriff basierend auf dem drahtlosen Berechtigungsnachweis zu empfangen; und
gemäß der empfangenen Berechtigung für den Zugriff ein Entriegeln der Lockbox zu veranlassen, um Benutzerzugriff auf eine Benutzerschnittstelle (4, 6) der Infusionspumpe und ein Medikamentenaufnahmeteil der Infusionspumpe zu ermöglichen, indem die mehrfach faltbaren Türen automatisch eingezogen werden, um zumindest einen Teil einer Vorderfläche der Infusionspumpe freizulegen, und die einziehbare Oberseite automatisch geöffnet wird, wodurch Benutzerzugriff auf zumindest einen Teil einer Oberseite der Infusionspumpe bereitgestellt wird.

2. System nach Anspruch 1, wobei der Prozessor ferner dazu ausgebildet ist:
über die Lockbox-Steuerschnittstelle einen Benutzerberechtigungsnachweis zu empfangen; und
über die Lockbox-Steuerschnittstelle den empfangenen Benutzerberechtigungsnachweis an die Pumpenkommunikationsschnittstelle zu übertragen, wobei die Berechtigung für den Zugriff von der Pumpenkommunikationsschnittstelle als Reaktion auf das Übertragen des Benutzerberechtigungsnachweises an die Pumpenkommunikationsschnittstelle von der Lockbox-Steuerschnittstelle empfangen wird.

3. System nach Anspruch 2, wobei die Lockbox-Steuerschnittstelle einen Scanner umfasst, der dazu ausgebildet ist, den Benutzerberechtigungsnachweis drahtlos von einem mit einem Benutzer verbundenen sendenden Gerät zu empfangen, wobei der Prozessor ferner dazu ausgebildet ist:
den Benutzerberechtigungsnachweis von einem durch den Scanner gescannten Ausweis zu empfangen.

4. System nach einem der Ansprüche 1-3, wobei das elektronische Koppeln der Lockbox-Steuerschnittstelle mit der Pumpenkommunikationsschnittstelle Folgendes umfasst:
drahtloses Koppeln der Lockbox-Steuerschnittstelle mit der Pumpenkommunikationsschnittstelle.

5. System nach Anspruch 1, wobei der Prozessor ferner dazu ausgebildet ist:
als Reaktion auf das Entriegeln der Lockbox die Benutzerschnittstelle der Infusionspumpe automatisch von einem Aus-Zustand in einen aktivierten Zustand umzuschalten.

6. System nach Anspruch 1, wobei die Lockbox ferner jeweilige linke und rechte Seiten (208a, 208b) umfasst, die dazu ausgebildet sind, sich in eine Richtung weg von der Vorderfläche des Gehäuses zu bewegen, wenn die Lockbox entriegelt ist, wobei der Prozessor ferner dazu ausgebildet ist:
als Reaktion auf das Entriegeln der Lockbox die jeweiligen linken und rechten Seiten automatisch zu bewegen, wodurch Benutzerzugriff auf zumindest einen Teil einer linken und rechten Seite der Infusionspumpe bereitgestellt wird.

7. System nach einem der Ansprüche 1-6, wobei die Lockbox-Steuerschnittstelle dazu ausgebildet ist, Benutzereingaben zu empfangen, wobei der Prozessor ferner dazu ausgebildet ist:
die Lockbox-Steuerschnittstelle zu aktivieren, um Benutzereingaben zu empfangen, wenn die Lockbox entriegelt ist;
die Benutzereingaben über die aktivierte Lockbox-Steuerschnittstelle zu empfangen; und
nach der Berechtigung für den Zugriff die Benutzereingaben an die Pumpenkommunikationsschnittstelle zur Bedienung der Infusionspumpe gemäß der Berechtigung für den Zugriff zu übertragen.

8. System nach einem der Ansprüche 1, wobei die Pumpenkommunikationsschnittstelle einen Scanner umfasst und dazu ausgebildet ist, einen Benutzerberechtigungsnachweis drahtlos über den Scanner von einem mit einem Benutzer verbundenen sendenden Gerät zu empfangen und die Berechtigung für den Zugriff basierend auf dem drahtlosen Berechtigungsnachweis an die Lockbox-Steuerschnittstelle zu übertragen.

9. Verfahren (400) zum Sichern einer Pumpe, umfassend:
Sichern (402) zumindest eines Teils einer Infusionspumpe innerhalb einer Lockbox, wobei die Lockbox ein Gehäuse umfasst, das eine Infusionspumpe einschließt, wobei das Gehäuse eine einziehbare, mehrfach faltbare Tür an jeder Seite einer Vorderfläche des Gehäuses umfasst, die, wenn sie geschlossen sind, eine Vorderfläche des Gehäuses bilden, und eine einziehbare Oberseite, die, wenn sie geschlossen ist, integral mit der Vorderfläche gekoppelt ist, wobei die Lockbox eine mit der Lockbox verbundene und innerhalb der Lockbox eingeschlossene Lockbox-Steuerschnittstelle umfasst, die dazu ausgebildet ist, eine Berechtigung für den Zugriff auf die Lockbox von einer Pumpenkommunikationsschnittstelle der Infusionspumpe zu erhalten;
elektronisches Koppeln (404) der Lockbox-Steuerschnittstelle mit der Pumpenkommunikationsschnittstelle;
Ermöglichen (406) der Übertragung eines drahtlosen Berechtigungsnachweises an die Pumpenkommunikationsschnittstelle zur Berechtigung für die Lockbox;
Empfangen (408), durch die Lockbox-Steuerschnittstelle von der Pumpenkommunikationsschnittstelle, der Berechtigung für den Zugriff basierend auf dem drahtlosen Berechtigungsnachweis; und
Veranlassen, durch die Lockbox-Steuerschnittstelle, gemäß der empfangenen Berechtigung für den Zugriff, eines Entriegelns der Lockbox, um Benutzerzugriff auf eine Benutzerschnittstelle der Infusionspumpe und ein Medikamentenaufnahmeteil der Infusionspumpe zu ermöglichen, indem die mehrfach faltbaren Türen automatisch eingezogen (414) werden, um zumindest einen Teil einer Vorderfläche der Infusionspumpe freizulegen, und die einziehbare Oberseite automatisch geöffnet (418) wird, wodurch Benutzerzugriff auf zumindest einen Teil einer Oberseite der Infusionspumpe bereitgestellt wird.

10. Verfahren nach Anspruch 9, ferner umfassend:
Empfangen eines Benutzerberechtigungsnachweises über die Lockbox-Steuerschnittstelle; und
Übertragen, durch die Lockbox-Steuerschnittstelle, des empfangenen Benutzerberechtigungsnachweises an die Pumpenkommunikationsschnittstelle, wobei die Berechtigung für den Zugriff von der Pumpenkommunikationsschnittstelle als Reaktion auf das Übertragen des Benutzerberechtigungsnachweises an die Pumpenkommunikationsschnittstelle von der Lockbox-Steuerschnittstelle empfangen wird.

11. Verfahren nach Anspruch 9, wobei die Pumpenkommunikationsschnittstelle einen Scanner umfasst, der dazu ausgebildet ist, einen Benutzerberechtigungsnachweis durch die Lockbox drahtlos von einem mit einem Benutzer außerhalb der Lockbox verbundenen sendenden Gerät zu empfangen, wobei die Berechtigung für den Zugriff von der Pumpenkommunikationsschnittstelle als Reaktion darauf, dass der Benutzerberechtigungsnachweis an der Pumpenkommunikationsschnittstelle gescannt wird, von der Lockbox-Steuerschnittstelle empfangen wird.

12. Verfahren nach einem der Ansprüche 9-11, wobei das elektronische Koppeln der Lockbox-Steuerschnittstelle mit der Pumpenkommunikationsschnittstelle Folgendes umfasst:
drahtloses Koppeln der Lockbox-Steuerschnittstelle mit der Pumpenkommunikationsschnittstelle.

13. Verfahren nach Anspruch 9, wobei das Verfahren ferner umfasst:
als Reaktion auf das Entriegeln der Lockbox die Benutzerschnittstelle der Infusionspumpe automatisch von einem Aus-Zustand in einen aktivierten Zustand umzuschalten.

14. Verfahren nach Anspruch 9, wobei die Lockbox ferner jeweilige linke und rechte Seiten umfasst, die dazu ausgebildet sind, sich in eine Richtung weg von der Vorderfläche des Gehäuses zu bewegen, wenn die Lockbox entriegelt ist, wobei das Verfahren ferner umfasst:
als Reaktion auf das Entriegeln der Lockbox die jeweiligen linken und rechten Seiten automatisch zu bewegen, wodurch Benutzerzugriff auf zumindest einen Teil einer linken und rechten Seite der Infusionspumpe bereitgestellt wird.

15. Verfahren nach einem der Ansprüche 9-14, wobei das Verfahren ferner umfasst:
Aktivieren der Lockbox-Steuerschnittstelle zum Empfangen von Benutzereingaben, wenn die Lockbox entriegelt ist;
Empfangen von Benutzereingaben über die aktivierte Lockbox-Steuerschnittstelle; und
nach der Berechtigung für den Zugriff Übertragen der Benutzereingaben an die Pumpenkommunikationsschnittstelle zur Bedienung der Infusionspumpe gemäß der Berechtigung für den Zugriff.

## Revendications

1. Système (500) destiné à sécuriser une pompe, comprenant:
un boîtier verrouillable (200) comprenant un logement renfermant une pompe d'infusion (30), le logement comprenant une porte rétractable à plis multiples (202a, 202b) de chaque côté d'une face avant du logement qui, lorsqu'elles sont fermées, forment une face avant du logement, et une partie supérieure rétractable (204) qui, lorsqu'elle est fermée, est couplée de manière intégrale à la face avant ; et
une interface de commande du boîtier verrouillable (214) associée au boîtier verrouillable et configurée pour obtenir une autorisation d'accès au boîtier verrouillable à partir d'une interface de communication de la pompe (52) de la pompe d'infusion et renfermée dans le boîtier verrouillable, l'interface de commande du boîtier verrouillable comprenant un processeur (50) configuré pour:
coupler électroniquement l'interface de commande du boîtier verrouillable à l'interface de communication de la pompe ;
faciliter la transmission d'un identifiant sans fil à l'interface de communication de la pompe pour l'autorisation du boîtier verrouillable ;
recevoir, depuis l'interface de communication de la pompe, l'autorisation d'accès basée sur l'identifiant sans fil ; et
provoquer, conformément à l'autorisation d'accès reçue, le déverrouillage du boîtier verrouillable afin de permettre à un utilisateur d'accéder à une interface utilisateur (4, 6) de la pompe d'infusion et à un réceptacle de médicament de la pompe d'infusion en rétractant automatiquement les portes à plis multiples afin d'exposer au moins une partie d'une face avant de la pompe d'infusion et en ouvrant automatiquement la partie supérieure rétractable, fournissant ainsi à l'utilisateur un accès à au moins une partie d'une surface supérieure de la pompe d'infusion.

2. Système selon la revendication 1, dans lequel le processeur est en outre configuré pour:
recevoir un identifiant utilisateur via l'interface de commande du boîtier verrouillable ; et
transmettre, via l'interface de commande du boîtier verrouillable, l'identifiant utilisateur reçu à l'interface de communication de la pompe, l'autorisation d'accès étant reçue par l'interface de commande du boîtier verrouillable depuis l'interface de communication de la pompe en réponse à la transmission de l'identifiant utilisateur à l'interface de communication de la pompe.

3. Système selon la revendication 2, dans lequel l'interface de commande du boîtier verrouillable comprend un scanner configuré pour recevoir l'identifiant utilisateur sans fil depuis un dispositif émetteur associé à un utilisateur, le processeur étant en outre configuré pour :
recevoir l'identifiant utilisateur depuis un badge scanné par le scanner.

4. Système selon l'une quelconque des revendications 1 à 3, dans lequel le couplage électronique de l'interface de commande du boîtier verrouillable à l'interface de communication de la pompe comprend:
apparier sans fil l'interface de commande du boîtier verrouillable à l'interface de communication de la pompe.

5. Système selon la revendication 1, dans lequel le processeur est en outre configuré pour :
commuter automatiquement, en réponse au déverrouillage du boîtier verrouillable, l'interface utilisateur de la pompe d'infusion d'un état éteint à un état activé.

6. Système selon la revendication 1, dans lequel le boîtier verrouillable comprend en outre des côtés gauche et droit respectifs (208a, 208b) configurés pour se déplacer dans une direction s'éloignant de la face avant du logement lorsque le boîtier verrouillable est déverrouillé, dans lequel le processeur est en outre configuré pour:
déplacer automatiquement, en réponse au déverrouillage du boîtier verrouillable, les côtés gauche et droit respectifs, fournissant ainsi à l'utilisateur un accès à au moins une partie d'un côté gauche et d'un côté droit de la pompe d'infusion.

7. Système selon l'une quelconque des revendications 1 à 6, dans lequel l'interface de commande du boîtier verrouillable est configurée pour recevoir une entrée utilisateur, dans lequel le processeur est en outre configuré pour :
activer l'interface de commande du boîtier verrouillable pour recevoir une entrée utilisateur lorsque le boîtier verrouillable est déverrouillé ;
recevoir l'entrée utilisateur via l'interface de commande du boîtier verrouillable activée ; et
transmettre, après l'autorisation d'accès, l'entrée utilisateur à l'interface de communication de la pompe pour le fonctionnement de la pompe d'infusion conformément à l'autorisation d'accès.

8. Système selon l'une quelconque des revendications 1, dans lequel l'interface de communication de la pompe comprend un scanner et est configurée pour recevoir un identifiant utilisateur sans fil via le scanner depuis un dispositif émetteur associé à un utilisateur, et pour transmettre l'autorisation d'accès basée sur l'identifiant sans fil à l'interface de commande du boîtier verrouillable.

9. Procédé (400) destiné à sécuriser une pompe, comprenant:
sécuriser (402) au moins une partie d'une pompe d'infusion dans un boîtier verrouillable, le boîtier verrouillable comprenant un logement renfermant une pompe d'infusion, le logement comprenant une porte rétractable à plis multiples de chaque côté d'une face avant du logement qui, lorsqu'elles sont fermées, forment une face avant du logement, et une partie supérieure rétractable qui, lorsqu'elle est fermée, est couplée de manière intégrale à la face avant, le boîtier verrouillable comprenant une interface de commande du boîtier verrouillable configurée pour obtenir une autorisation d'accès au boîtier verrouillable à partir d'une interface de communication de la pompe de la pompe d'infusion et renfermée dans le boîtier verrouillable ;
coupler électroniquement (404) l'interface de commande du boîtier verrouillable à l'interface de communication de la pompe ;
faciliter (406) la transmission d'un identifiant sans fil à l'interface de communication de la pompe pour l'autorisation du boîtier verrouillable ;
recevoir (408), par l'interface de commande du boîtier verrouillable depuis l'interface de communication de la pompe, l'autorisation d'accès basée sur l'identifiant sans fil ; et
provoquer, par l'interface de commande du boîtier verrouillable, conformément à l'autorisation d'accès reçue, le déverrouillage du boîtier verrouillable afin de permettre à un utilisateur d'accéder à une interface utilisateur de la pompe d'infusion et à un réceptacle de médicament de la pompe d'infusion en rétractant automatiquement (414) les portes à plis multiples afin d'exposer au moins une partie d'une face avant de la pompe d'infusion et en ouvrant automatiquement (418) la partie supérieure rétractable, fournissant ainsi à l'utilisateur un accès à au moins une partie d'une surface supérieure de la pompe d'infusion.

10. Procédé selon la revendication 9, comprenant en outre :
recevoir un identifiant utilisateur via l'interface de commande du boîtier verrouillable ; et
transmettre, par l'interface de commande du boîtier verrouillable, l'identifiant utilisateur reçu à l'interface de communication de la pompe, l'autorisation d'accès étant reçue par l'interface de commande du boîtier verrouillable depuis l'interface de communication de la pompe en réponse à la transmission de l'identifiant utilisateur à l'interface de communication de la pompe.

11. Procédé selon la revendication 9, dans lequel l'interface de communication de la pompe comprend un scanner configuré pour recevoir un identifiant utilisateur à travers le boîtier verrouillable sans fil depuis un dispositif émetteur associé à un utilisateur à l'extérieur du boîtier verrouillable, dans lequel l'autorisation d'accès est reçue par l'interface de commande du boîtier verrouillable depuis l'interface de communication de la pompe en réponse au scan de l'identifiant utilisateur au niveau de l'interface de communication de la pompe.

12. Procédé selon l'une quelconque des revendications 9 à 11, dans lequel le couplage électronique de l'interface de commande du boîtier verrouillable à l'interface de communication de la pompe comprend :
apparier sans fil l'interface de commande du boîtier verrouillable à l'interface de communication de la pompe.

13. Procédé selon la revendication 9, le procédé comprenant en outre :
commuter automatiquement, en réponse au déverrouillage du boîtier verrouillable, l'interface utilisateur de la pompe d'infusion d'un état éteint à un état activé.

14. Procédé selon la revendication 9, dans lequel le boîtier verrouillable comprend en outre des côtés gauche et droit respectifs configurés pour se déplacer dans une direction s'éloignant de la face avant du logement lorsque le boîtier verrouillable est déverrouillé, le procédé comprenant en outre :
déplacer automatiquement, en réponse au déverrouillage du boîtier verrouillable, les côtés gauche et droit respectifs, fournissant ainsi à l'utilisateur un accès à au moins une partie d'un côté gauche et d'un côté droit de la pompe d'infusion.

15. Procédé selon l'une quelconque des revendications 9 à 14, dans lequel le procédé comprend en outre :
activer l'interface de commande du boîtier verrouillable pour recevoir une entrée utilisateur lorsque le boîtier verrouillable est déverrouillé ;
recevoir une entrée utilisateur via l'interface de commande du boîtier verrouillable activée ; et
transmettre, après l'autorisation d'accès, l'entrée utilisateur à l'interface de communication de la pompe pour le fonctionnement de la pompe d'infusion conformément à l'autorisation d'accès.
